(19) Europäisches Patentamt
European Patent Office
Office européen des brevets

(11) **EP 1 836 497 B1**

(12) **EUROPEAN PATENT SPECIFICATION**

(45) Date of publication and mention
of the grant of the patent:
**03.04.2013 Bulletin 2013/14**

(51) Int Cl.:
***G01N 33/543*** *(2006.01)*     ***G01N 31/16*** *(2006.01)*
***G01N 33/58*** *(2006.01)*

(21) Application number: **05854539.3**

(22) Date of filing: **16.12.2005**

(86) International application number:
**PCT/US2005/045846**

(87) International publication number:
**WO 2006/066163 (22.06.2006 Gazette 2006/25)**

(54) **QUANTUM DOT-ENCODED BEAD SET FOR CALIBRATION AND QUANTIFICATION OF MULTIPLEXED ASSAYS, AND METHODS FOR THEIR USE**

QUANTENPUNKT-CODIERTER KÜGELCHENSATZ ZUR KALIBRIERUNG UND QUANTIFIZIERUNG VON MULTIPLEX-TESTS SOWIE VERFAHREN ZUR VERWENDUNG DAVON

ENSEMBLE BILLES CODEES PAR DES POINTS QUANTIQUES POUR ETALONNER ET QUANTIFIER DES DOSAGES MULTIPLES, ET PROCEDES D'UTILISATION DE CELLES-CI

(84) Designated Contracting States:
**AT BE BG CH CY CZ DE DK EE ES FI FR GB GR HU IE IS IT LI LT LU LV MC NL PL PT RO SE SI SK TR**

(30) Priority: **16.12.2004 US 637347 P**

(43) Date of publication of application:
**26.09.2007 Bulletin 2007/39**

(73) Proprietor: **Life Technologies Corporation**
**Carlsbad, CA 92008 (US)**

(72) Inventors:
• **EASTMAN, Paul, Scott**
**Danville, CA 94526 (US)**
• **NUTTALL, Rachel, L.**
**San Jose, CA 95112 (US)**
• **DOCTOLERO, Michael H.**
**Oakland, CA 94606 (US)**

(74) Representative: **HOFFMANN EITLE**
**Patent- und Rechtsanwälte**
**Arabellastrasse 4**
**81925 München (DE)**

(56) References cited:
**US-A- 5 981 180**     **US-A1- 2002 187 515**
**US-A1- 2003 165 951**

**Description**

FIELD OF THE INVENTION

The invention relates to the use of control beads to improve the quantitative results of multiplexed bead-based assays.

DESCRIPTION OF RELATED ART

Many multiplexed assays exist to facilitate the rapid screening and measurement of large numbers of analytes simultaneously. These assays have enabled chemical, biological, and biomedical researchers to easily screen libraries of analytes, where individual screening of the library members would be prohibitively costly both in time and resources.

For example, US 2002/187515 A1 describes sensor compositions comprising a composite array of individual arrays, to allow for simultaneous processing of a number of samples. The composite array compositions comprise a first substrate with a surface comprising a plurality of assay locations, each assay location comprising a plurality of discrete sites. The substrate further comprises a population of microspheres comprising at least a first and a second subpopulation, wherein each subpopulation comprises a bioactive agent.

US 2003/165951 A1 describes methods, compositions and articles of manufacture for assaying a sample for a target polynucleotide and/or an amplification product therefrom are provided. The methods comprise contacting a sample suspected of containing the target polynucleotide with a polynucleotide that can bind specifically thereto; this polynucleotide is conjugated to a substrate, preferably an encoded bead conjugate. An amplification reaction can first be used to produce the amplification product from the target polynucleotide so that it can be used to indirectly assay for the target polynucleotide.

The most well known multiplexed assay format is the two-dimensional array. In essence, a two dimensional grid of materials is formed on a chip using photolithography or other physical methods. A sample suspected of containing one or more analytes is allowed to contact the chip, and bound analytes are detected. This assay format has been commercialized by Affymetrix and others.

An alternative multiplexed assay format is based on flow cytometry to form a "liquid array". Beads are individually labeled with specific ratios of multiple dyes, and allowed to interact with targets that are linked to a "reporter" linked to the target of interest. The beads are analyzed individually using a flow cytometry system. This assay format has been commercialized by Luminex, Bio-Rad Laboratories, Qiagen, and others.

Despite the usefulness of the various commercial multiplexing systems, they all suffer from the problem of compressed dynamic ranges. Essentially, the dynamic range of the signal output from interaction with a desired target is compressed relative to the dynamic range of the target input. This compressed dynamic range greatly compromises accurate quantitation of the target analyte. Thus, there exists a need for new or improved multiplexed assay methods that address this problem and deliver improved quantitative results.

SUMMARY OF THE INVENTION

The present invention concerns a method of determining the concentration of at least one analyte in a multiplexed assay as defined by claims 1 to 20. The method uses a set of control beads containing a range of calibration moieties. The control beads are combined with sample beads to allow the formation of a titration curve. Use of the titration curve improves the determination of analyte concentration from the sample beads in a multiplexed assay.

DESCRIPTION OF THE FIGURES

The following figures form part of the present specification and are included to further demonstrate certain aspects of the present invention. The invention may be better understood by reference to one or more of these figures in combination with the detailed description of specific embodiments presented herein.

Figure 1 shows the plot of best-fit models against raw data from Example 6.

Figure 2 shows the residuals from the best-fit models.

Figure 3 shows a comparison of second order, third order, fourth order, and sigmoidal fits residuals.

Figure 4 shows a comparison of best-fit models using a one-sided t-test.

Figure 5 shows a plot of $Log_{10}$[Raw RFU] (y-axis) against $Log_{10}$[biotin/bead] (x-axis).

Figure 6 shows a plot of $Log_{10}$[Calibrated RFU] (y-axis) against the $Log_{10}$[biotin/bead] (x-axis).

Figure 7 shows a plot of $Log_{10}$[Raw RFU] (y-axis) against $Log_{10}$[molecule input] (x-axis) for hybridization control beads.

Figure 8 shows a plot of $Log_{10}$[Calibrated RFU; biotins per bead] (y-axis) against $Log_{10}$[molecule input] (x-axis) for hybridization control beads.

Figure 9 shows a plot of $Log_{10}$[Raw RFU] against $Log_{10}$[molecule input] for RNA spike samples.

Figure 10 shows a plot of $Log_{10}$[Calibrated RFU; biotins per bead] against $Log_{10}$[molecule input] for RNA spike samples.

Figure 11 shows the expression level of endogenous genes using raw data.

Figure 12 shows the expression level of endogenous genes using a biotin density titration curve to calibrate the raw data.

DETAILED DESCRIPTION OF THE INVENTION

While compositions and methods are described in terms of "comprising" various components or steps (interpreted as meaning "including, but not limited to"), the compositions and methods can also "consist essentially of" or "consist of the various components and steps, such terminology should be interpreted as defining essentially closed-member groups.

Aspects of the instant disclosure involve the preparation and use of a set of control beads in multiplexed assays. The control beads provide for the preparation of a titration curve in the claimed methods, thereby improving the quality of the quantitative measurement of analytes of interest. The term "a bead" can refer to a single bead, or more commonly to a single type of bead. In actual laboratory assays, hundreds or thousands of a particular type of bead would be used, either alone or as a mixture of multiple types of beads. Small spherical beads are sometimes referred to in the art as "microspheres".

Materials - control beads

One embodiment of the disclosure is directed towards a set of control beads. The set comprises a plurality of beads comprising a coding moiety and a calibration moiety, wherein the coding moiety is present at a fixed concentration in the plurality of beads; and the calibration moiety is present at a range of concentrations in the plurality of beads. The coding moiety can be inside the beads, distributed throughout the beads, or arrayed at or near the surface of the beads. The calibration moiety can be inside the beads, distributed throughout the beads, or arrayed at or near the surface of the beads. The range of concentrations can be from low to high concentration, or phrased differently, the beads can have a range from low to high of absolute numbers of calibration moieties on the beads. The control beads can comprise more than one coding moiety, for example, 2, 3, 4, 5, 6, and so on. The control beads can comprise more than one calibration moiety, for example, 2, 3, 4, 5, 6, and so on.

The beads can generally be any type of beads. For example, the beads can be polymer beads. Examples of polymer beads include polystyrene beads, poly(ethyl methacrylate)) beads, poly(methyl methacrylate) beads, polyacrylate beads, dextran beads, melamine particles crosslinked by acid catalyzed reaction with formaldehyde, polyactide beads, and poly(e-caprolectone) beads. Alternatively, the beads can be glass, silica, ceramic, zirconia, titania, alumina, gold, silver, palladium, or platinum beads. The beads can have additional properties such as being paramagnetic or being dispersable in water. While beads are commonly spherical in shape, they are not required to be so, and can be other shapes such as rod-shaped, oblong, or irregular in shape.

For spherical beads, the bead can generally have any diameter. Examples of diameters include about 0.05 $\mu$m, about 0.1 $\mu$m, about 0.5 $\mu$m, about 1 $\mu$m, about 2 $\mu$m, about 3 $\mu$m, about 4 $\mu$m, about 5 $\mu$m, about 6 $\mu$m, about 7 $\mu$m, about 8 $\mu$m, about 9 $\mu$m, about 10 $\mu$m, about 11 $\mu$m, about 12 $\mu$m, about 13 $\mu$m, about 14 $\mu$m, about 15 $\mu$m, about 20 $\mu$m, about 30 $\mu$m, about 40 $\mu$m, about 50 $\mu$m, about 60 $\mu$m, about 70 $\mu$m, about 80 $\mu$m, about 90 $\mu$m, about 100 $\mu$m, and ranges between any two of these values. It is typical, but not required, that the beads all have about the same size.

The coding moiety and the calibration moiety can be associated with the beads in a variety of manners. The coding moiety and the calibration moiety can be associated with the beads in the same manner, or in different manners. For example, the coding moiety and the calibration moiety can be covalently bound to the beads, electrostatically bound to the beads, associated with the beads by pi-pi interactions, associated with the beads by van der waals interactions, physically entrapped within the beads or the bead surface, and so on. The coding moiety and the calibration moiety can be directly associated with the beads, or can be indirectly associated via a linker or other intermediary.

The coding moiety and the calibration moiety can generally be any detectable moieties. The coding moiety and the calibration moiety can be the same or different, but typically are different. The detectable moieties can be directly detectable or indirectly detectable. Directly detectable moieties can be detected without addition of another molecule. Examples of directly detectable moieties include semiconductor nanocrystals, fluorescent organic dyes, fluorescent proteins, radioactive isotopes, and so on. Directly detectable moieties can alternatively be physical such as an etched code or a RFID (radio frequency identification) chip. An indirectly detectable moiety can have several parts or pieces. An example of such could be a first binding partner and a second binding partner which together are detectable. Examples of indirectly detectable moieties include enzymes that cause a chemical reaction that releases a detectable product, such as cleavage of a strained precursor which releases light or color (such as a beta-lactamase enzyme to release color, or a peroxidase or phosphatase enzyme to release light).

In a presently preferred embodiment, the coding moiety is directly detectable, and the calibration moiety is indirectly detectable. It is presently preferred that the coding moiety is one or more semiconductor nanocrystals.

A specific example of a set of beads are beads having one or more semiconductor nanocrystals embedded, impregnated, or otherwise associated with the beads. These semiconductor nanocrystals can be a coding moiety. The beads can also have at least one biotinylated compound at their surface, this being a calibration moiety. The biotinylated compound can interact with a labeled avidin or streptavidin protein. The label on the avidin or streptavidin protein can include semiconductor nanocrystals, fluorescent organic dyes, radioactive isotopes, and so on. A more specific example are beads having one or more first semiconductor nanocrystals embedded, impregnated, or otherwise associated with the beads (a coding moiety), at least one biotinylated oligonucleotide attached to the surface of the beads (a calibration moiety). The biotinylated oligonucleotide can be detected by use of streptavidin labeled with a second semiconductor nanocrystal. It is preferred that the first semiconductor nanocrystal(s) and the second semiconductor nanocrystal emit light of different wavelengths so as to be independently detectable. The set of beads can have the same biotinylated oligonucleotide at their surface, but in different concentrations so as to generate a range of signals from different beads Many other alternatives to biotin exist, such as a hapten with a corresponding antibody, DIG with an anti-DIG antibody, dinitrophenol with an anti-DNP antibody, a metal chelator such as a polyhistidine tag with nickel NTA, a SH2 domain, and so on. Chemical reactions can also be used to associate a labeled entity with the beads. Such chemical reactions can include amines with NHS esters, amino acid coupling chemistries, and so on.

The signal generated by the set of beads preferably has a wide range to facilitate the preparation of a titration curve. The signal can be generated by a wide variety of methods. For example, the signal can be generated by addition of visible light, UV light, illumination with a laser, illumination with a laser diode, illumination with an LED, and so on. The ratio of signal from the bead having the highest concentration of calibration moiety to the signal from the bead having the lowest concentration of calibration moiety is preferably greater than 1, greater than about 1.1, greater than about 1.2, greater than about 1.3, greater than about 1.4, greater than about 1.5, greater than about 1.6, greater than about 1.7, greater than about 1.8, greater than about 1.9, greater than about 2, greater than about 3, greater than about 4, greater than about 5, greater than about 6, greater than about 7, greater than about 8, greater than about 9, greater than about 10, greater than about 100, greater than about 1,000, greater than about $10^4$, greater than about **$10^5$,** greater than about $10^6$, greater than about $10^7$, greater than about $10^8$, greater than about $10^9$, greater than about $10^{10}$, greater than about $10^{11}$, greater than about $10^{12}$, and ranges between any two of these values.

Materials - bead mixtures

An additional embodiment of the disclosure is directed towards compositions comprising a set of sample beads configured to interact with an array of analytes, and a set of control beads configured to allow preparation of a titration curve, in the claimed methods.

The set of sample beads can be configured to bind to analytes such as DNA, RNA, PNA, proteins, antibodies, ligands, receptors, lipids, polysaccharides, and so on. The analytes can be biotinylated or unbiotinylated. The set of sample beads can have an array of capture moieties on their surface that allow selective binding of a specific analyte. For example, a sample bead or beads could have a first oligonucleotide on its surface that allows specific hybridization with a sequence complementary with the first oligonucleotide; a second bead or beads could have a second oligonucleotide on its surface that allows specific hybridization with a sequence complementary with the second oligonucleotide, and so on. The capture moiety can generally be any material or compound configured to selectively bind the analyte of interest. Examples of capture moieties include DNA, RNA, peptides, proteins, antibodies, and so on. The set of sample beads can comprise a coding moiety, or two or more coding moieties. In a presently preferred embodiment, the set of sample beads comprise a coding moiety and a capture moiety. The set of sample beads can have one or more semiconductor nanocrystals embedded, impregnated, or otherwise associated with the beads as a coding moiety or moieties.

The second set of beads can be any of the set of control beads described above in the previous section.

A specific example of a bead mixture is a set of sample beads having one or more semiconductor nanocrystals embedded, impregnated, or otherwise associated with the beads (a first coding moiety), and an array of oligonucleotides on their surface (a first capture moiety or moieties); and a set of control beads having one or more semiconductor nanocrystals embedded, impregnated, or otherwise associated with the beads (a second coding moiety), and having biotinylated oligonucleotides on their surface (a calibration moiety).

Kits

An additional embodiment of the disclosure is directed towards kits containing bead compositions. The kit can comprise a protocol for obtaining a titration curve, and any of the bead mixtures described above in the previous section.

Methods of use in preparing a titration curve

An additional embodiment of the disclosure is directed towards a method of preparing a titration curve using the above described control beads. The method can comprise providing a set of control beads, wherein the set of control beads comprise a coding moiety and a calibration moiety; obtaining signal from the coding moiety, obtaining signal from the calibration moiety; and preparing a titration curve from the signal obtained from the calibration moiety. The set of control beads can have a range of concentrations of the calibration moiety.

The preparing a titration curve step can comprise analyzing the signal obtained from the calibration moiety using generally any method. The method can involve use of a linear first order polynomial, a second order polynomial, a third order polynomial, a fourth order polynomial, or a sigmoidal fit. It is presently preferred that the titration curve be obtained by use of a third order polynomial, a fourth order polynomial, or a sigmoidal fit.

Once the titration curve has been obtained, subsequent multiplexed analyte assays can be performed without generating a new titration curve. Alternatively, the titration curve can be obtained concurrently with the multiplexed analyte assays by methods such as simultaneous imaging or flow cytometry.

The coding moiety can generally be any coding moiety. The coding moiety can be a directly detectable moiety or an indirectly detectable moiety. It is presently preferred that the coding moiety is at least one semiconductor nanocrystal. If multiple semiconductor nanocrystals are used, they can generate a "barcode".

The calibration moiety can generally be any detectable moiety. The calibration moiety can be a directly detectable moiety or an indirectly detectable moiety. The calibration moiety can be a hapten. It is presently preferred that the calibration moiety is a biotinylated compound. It is also presently preferred that the calibration moiety is a biotinylated oligonucleotide. The biotinylated compound can be readily detected by addition of a labeled avidin or streptavidin, or other material that binds to the calibration moiety.

The method can further comprise contacting the set with labeled avidin or labeled streptavidin before obtaining signals. More particularly, the method can further comprise contacting the mixture with streptavidin labeled with at least one semiconductor nanocrystal before obtaining signals.

Methods of use in a multiplexed analyte assay

The above described control beads, bead mixtures, and kits can be used to improve the quantitative results of a multiplexed analyte assay. The use of the set of control beads allow for the preparation of a titration curve that can subsequently be used to quantify the concentration of one or more analytes. The instant inventors found that signal and concentrations of analytes did not directly correlate without use of the titration curve. Accordingly, the instant methods were found to greatly improve the accuracy of multiplexed analyte assays. The analyte can generally be any analyte. Examples of analytes include DNA, RNA, PNA, proteins, antibodies, ligands, receptors, lipids, polysaccharides, and so on. The analytes can be biotinylated or unbiotinylated.

The claimed invention is a method of determining the concentration of at least one analyte in a multiplexed assay, the method comprising: providing a mixture comprising a set of sample beads and a set of control beads, wherein the set of sample beads comprise a first coding moiety and a first capture moiety that selectively binds to the at least one analyte, and wherein the set of control beads comprise a second coding moiety at a fixed concentration in the plurality of beads and a calibration moiety present at a rang of concentrations in the plurality of beads, contacting the mixture with a sample suspected of containing the at least one analyte; obtaining signal from the first coding moiety, obtaining signal from the second coding moiety, obtaining signal from the calibration moiety; preparing a titration curve from the signal obtained from the calibration moiety; and using the titration curve and the image to determine the concentration of the at least one analyte. Obtaining the various signals can be performed serially (as in a flow cytometer, for example) or simultaneously (as by simultaneously imaging an array of beads, for example).

The preparing a titration curve step can comprise analyzing the signal obtained from the calibration moiety using generally any method. The method can involve use of a linear first order polynomial, a second order polynomial, a third order polynomial, a fourth order polynomial, or a sigmoidal fit. It is presently preferred that the titration curve be obtained by use of a third order polynomial, a fourth order polynomial, or a sigmoidal fit.

Once the titration curve has been obtained, subsequent multiplexed analyte assays can be performed without generating a new titration curve. Alternatively, the titration curve can be obtained concurrently with the multiplexed analyte assays by methods such as simultaneous imaging or flow cytometry.

The first coding moiety can generally be any coding moiety. The first coding moiety can be a directly detectable moiety or an indirectly detectable moiety. It is presently preferred that the first coding moiety is at least one semiconductor nanocrystal. If multiple semiconductor nanocrystals are used, they can generate a "barcode".

The second coding moiety can generally be any coding moiety. The second coding moiety can be a directly detectable moiety or an indirectly detectable moiety. It is presently preferred that the second coding moiety is at least one semiconductor nanocrystal. If multiple semiconductor nanocrystals are used, they can generate a "barcode".

The first coding moiety and the second coding moiety can be the same or different. It is presently preferred that the first coding moiety and the second coding moiety are different, in order to facilitate identification of the first set of beads and the second set of beads. It is possible, however, to have the first coding moiety and the second coding moiety to be the same compound(s)/material(s), but present in different concentrations.

The calibration moiety can generally be any detectable moiety. The calibration moiety can be a directly detectable moiety or an indirectly detectable moiety. The calibration moiety can be a hapten. It is presently preferred that the calibration moiety is a biotinylated compound. It is also presently preferred that the calibration moiety is a biotinylated oligonucleotide. The biotinylated compound can be readily detected by addition of a labeled avidin or streptavidin, or other material that binds to the calibration moiety.

The method can further comprise contacting the mixture with labeled avidin or labeled streptavidin before obtaining the signals. More particularly, the method can further comprise contacting the mixture with streptavidin labeled with at least one semiconductor nanocrystal before obtaining the signals.

The following examples are included to demonstrate preferred embodiments of the disclosure.

EXAMPLES

Example 1: Preparation of quantum dot nanoparticles

The preparation of quantum dot nanoparticles is well known in the art. Exemplary methods of preparing quantum dots are described in U.S. Patent Nos. 6,207,299, 6,322,901 and 6,576,291, and in the publication "Alternative Routes toward High Quality CdSe Nanocrystals," (Qu et al., Nano Lett., 1(6):333-337 (2001)). Quantum dot nanoparticles are commercially available from Quantum Dot Corporation (Hayward, CA). The preparation of quantum dot nanoparticles has been the subject of many patents and publications, the following are several examples. The use of alloyed or mixed shells has been described in U.S. Patent No. 6,815,064. The use of a promoter to make quantum dot cores has been described in U.S. Patent Publication No. 2003/0097976 (published May 29, 2003). Surface modification methods in which mixed hydrophobic/hydrophilic polymer transfer agents are bound to the surface of the quantum dots are suggested in U.S. Patent No. 6,649,139.

Example 2: Preparation of quantum dot nanoparticle impregnated microspheres

Methods for making quantum dot nanoparticle impregnated microspheres are well-known in the art. The preparation of quantum dot nanoparticle impregnated microspheres has been the subject of many patents and publications, the following are several examples. U.S. Patent No. 6,479,146 describes methods using electrostatic self-assembly of nanocomposite multilayers on decomposable colloidal templates. International Publication No. WO 00/77281 (published December 21, 2000) described encapsulation of crystals via multilayer coatings. International Publication No. WO 01/51196 (published July 19, 2001) described the templating of solid particles using polymer multilayers. International Publication No. WO 99/47252 (published September 23, 1999) described the use of layer-wise polyelectrolyte self-assembly to prepare nanocapsules and microcapsules. U.S. Patent Nos. 6,548,171 B1 and 6,680,211 B2 describe microspheres with embedded fluorescent nanocrystals. Finally, polyelectrolyte multilayer films were modeled by Park et al., Langmuir 18: 9600-9604 (2002).

Example 3: Preparation of binding calibration beads

The following protocol was used to prepare beads incorporating approximately equal quantities of orange and red emitting quantum dot nanoparticles. The beads can be conveniently separated from suspension either by bench-top centrifugation or magnetic separation during the wash steps and deposition steps. Spherical polystyrene base beads (9 $\mu$m, paramagnetic core, underivatized) were purchased from Polymer Laboratories (Shropshire, UK). The base beads (20 mg, 500 $\mu$L as a 4% solution) were washed with water and then incubated with poly-ethyleneimine (10 mL, 4%, about 25,000 g/mol MW, Aldrich Chemical (St. Louis, MO)) for 30 minutes. The beads were washed ten times by agitation in water followed by centrifugal separation and re-suspension.

Following the wash steps, the beads were further incubated with amphophilic polymer-solubilized CdSe/CdZnS core-shell quantum dot nanoparticles emitting at 591 nm (68.07 $\mu$L, 8 $\mu$M) and similarly prepared quantum dot nanoparticles emitting at 655 nm (40.23 $\mu$L, 8 $\mu$M) in 3.4 mL water for one hour with agitation. The quantum dot nanoparticle incubation was followed by another series of water washes (10 times), and re-suspended in a 4% poly-acrylic acid (about 1,200 g/mol MW, Aldrich Chemical (St. Louis, MO)) for 30 minutes. Following an additional 10 water washes, the beads were re-suspended in a 4% poly-ethyleneimine solution for 30 minutes. The beads were washed ten times and stored in 2 mL 1% poly-acrylic acid.

Example 4: Coniugation of biotinylated oligonucleotides to beads

Using the values in Tables 1 and 2 below, a stock mixture of biotinylated and non-biotinylated oligonucleotides, each 5' end-capped with primary amine functionality, was prepared in MES buffer (2-morpholinoethanesulfonic acid, 100 mM) to a final total oligonucleotide concentration of 2.5 $\mu$M. An EDC ((1-ethyl-3-[3-dimethylaminopropyl]carbodiimide hydrochloride)) stock solution was prepared in MES at a concentration of 4% by combining EDC (1 g) with 25 mL MES buffer (100 mM). Quantum dot nanoparticle impregnated beads (from previous Example; 1 million beads per reaction) were washed with water (3 times), and then 100 mM MES buffer (1 time) and re-suspended in MES (10 $\mu$L). Oligonucleotide stock solution (80 $\mu$L) was combined with the bead suspension along with EDC stock solution (90 $\mu$L) such that the final EDC concentration was 2%. The resulting reaction suspension was mixed and then allowed to react using a Vortemp shaking incubator overnight at 1500 rpm and at 22 °C. Following the reaction, the beads were washed and stored in 500 $\mu$L PBS at 4 C.

Table 1

| % bt oligo | pmoles bt oligo per 80 uL | biotin per bead (pmoles x 1e-12 x 6.02e-23 / 1,000,000 x 0.2 |
|---|---|---|
| 100 | 200 | 24,080,000 |
| 20 | 40 | 4,816,000 |
| 4 | 8 | 963,200 |
| 0.8 | 1.6 | 192.640 |
| 0.16 | 0.32 | 38,528 |
| 0.032 | 0.064 | 7,706 |
| 0.0064 | 0.0128 | 1,541 |
| 0.00128 | 0.00256 | 308 |
| 0.000256 | 0.000512 | 62 |

Table 2

| Dilution tube | uL non-bt oligo added | uL of bt oligo added | uL removed after mixing (added to next tube) | Total uL after dilution | %bt oligo in tube after dilution |
|---|---|---|---|---|---|
| 1 | 0 | 125 | 25 | 100 | 100 |
| 2 | 100 | 0 | 25 | 100 | 20 |
| 3 | 100 | 0 | 25 | 100 | 4 |
| 4 | 100 | 0 | 25 | 100 | 0.8 |
| 5 | 100 | 0 | 25 | 100 | 0.16 |
| 6 | 100 | 0 | 25 | 100 | 0.03 |
| 7 | 100 | 0 | 25 | 100 | 0.006 |
| 8 | 100 | 0 | 25 | 100 | 0.0013 |
| 9 | 100 | 0 | 25 | 100 | 0.00026 |

Example 5: Assay and hybridization protocol

Gene panel selection, e.g., 50 target genes from an appropriate sample (e.g., cell, tissue, etc.) were selected for analysis. One capture probe was designed and selected for each target gene. Each capture probe was conjugated to a population of uniquely optically detectable quantum dot nanoparticle-impregnated beads. The target genes and spiked oligonucleotides were amplified and labeled with biotin using the T7 amplification methods disclosed in U.S. Patent Nos. 5,514,545; 5,545,522; 5,716,785; and 5,891,636.

The following three components were added to each well of a 96-well microtiter plate:

(a) for the given gene panel having, e.g., 50 members, one capture probe for each gene conjugated to one of 50 uniquely encoded beads;

(b) for a given panel, e.g., 20 members, of predetermined housekeeping genes, there will be a corresponding set of capture probes that were conjugated to an additional set of 20 uniquely encoded beads added to each well; and

(c) an additional set of 20 uniquely encoded beads that serve as various controls added to each well.

Binding calibration beads

The binding calibration beads are a series of, e.g., 9, uniquely encoded beads added to each well with increasing levels of biotin-oligo attached, prepared as described in an earlier Example. A binding calibration curve is generated for each well from the signal obtained from the series. This data provides information on the signal level (RFU) generated by a known number of biotins per bead. This data is then used to calibrate the signal from all other beads in each well. Thus, providing a "Calibrated RFU" or the numbers of biotins per bead for each uniquely encoded bead in each well. The Calibrated RFU is used to quantitate the number of target molecules using methods such as those shown in the Data Curve Fitting algorithm provided in the next Example.

Hybridization control beads

An additional set of beads that can be used are hybridization control beads. These beads have one or more different defined oligonucleotide sequences on their surface. A biotinylated reverse complement oligonucleotide can be bound in a dilution series, testing the hybridization effectiveness of the system. The oligonucleotide sequences can be designed to be unique and to not cross-hybridize with any target sequence of interest. Nonspecific hybridization control beads can also be used, having oligonucleotide sequences designed to not hybridize to any known sequence in the assay.

The Calibrated RFU from the hybridization control beads is plotted versus the copy number to provide information about, e.g., whether the signal level generated is acceptable, whether the hybridization step of the protocol was effective, whether the reaction is linear, and how high the background signal is. The nonspecific hybridization control yields information about the level of nonspecific hybridization.

Labeling Control Beads

Labeling control beads can be used to act as a form of positive control. Synthetic RNA transcripts of known sequence can be added to the sample prior to contacting with the beads. The synthetic RNA transcripts can be added at different concentrations to test the response of the labeling control beads. Labeling control beads can be added, having oligonucleotide sequences designed to hybridize with the amplified transcript sequence. The synthetic RNA transcripts and their complements are designed to not be cross-reactive with other analyte sequences.

A "Calibrated RFU" is generated for each of the Labeling Control Beads. The Labeling Control Beads Calibrated RFUs are plotted against the known concentration of "spike" that is added to the Sample Labeling and Spike Labeling step. These data provide information about, e.g., whether the signal level generated is acceptable, whether the amplification/labeling step of the protocol was effective, whether the labeling reaction is linear, and how high the background signal is.

Instrument control beads

An Instrument Control Bead can also be added to each well. This bead comprises a bead dyed with a reporter quantum dot nanocrystal, e.g., the 655 nm peak emission quantum dot. The Instrument Control Bead can have multiple reporter colors, e.g. 655 nm and 705 nm.

Typically, the following beads are added to each well of an assay for a 50-plex (i.e., the simultaneous assay of the expression of 50 unique target genes). Table 3 gives an example set of beads, but the numbers of each of the beads, and the total number of beads can be varied. Each bead identity is present in a well at a large number, with the total number of beads in the well being in the thousands.

Table 3

| Type | Number of bead identities |
|---|---|
| 1 code for each of the genes selected as a target | 50 beads |
| 1 code for each of 20 housekeeping genes | 20 beads |
| Control beads | 20 beads total |
| Instrument control bead | 1 bead |
| Binding control beads | 9 beads |
| Hybridization control bead | 6 beads |
| Labeling control beads | 4 beads |

Example 6: Data curve fitting algorithm

The most appropriate data model (curve fit) for measured intensity as a function of biotin per bead was determined.

The residuals from fitting a second, third, and fourth order polynomial as well as sigmoidal curve were calculated and compared via Student's t-test The results indicate that the sigmoidal, third order polynomial, and fourth order polynomial fits were all significantly better than the second order polynomial. The fourth order polynomial fit was not significantly better than the third order polynomial or sigmoidal fit. The sigmoidal fit and the third order polynomial fit were nearly equivalent in describing the experimental data. The Biotin density for each OligoID label was assigned from Table 4.

Table 4

| Oligo ID | Biotin molecules per bead |
|---|---|
| Binding control bead 1 | 24,080,000 |

(continued)

| Oligo ID | Biotin molecules per bead |
|---|---|
| Binding control bead 2 | 6,020,000 |
| Binding control bead 3 | 1,505,000 |
| Binding control bead 4 | 376,250 |
| Binding control bead 5 | 94,063 |
| Binding control bead 6 | 23,516 |
| Binding control bead 7 | 5,879 |
| Binding control bead 8 | 1,140 |
| Binding control bead 9 | 367 |
| Binding control bead 10 | 92 |
| Binding control bead 11 | 23 |

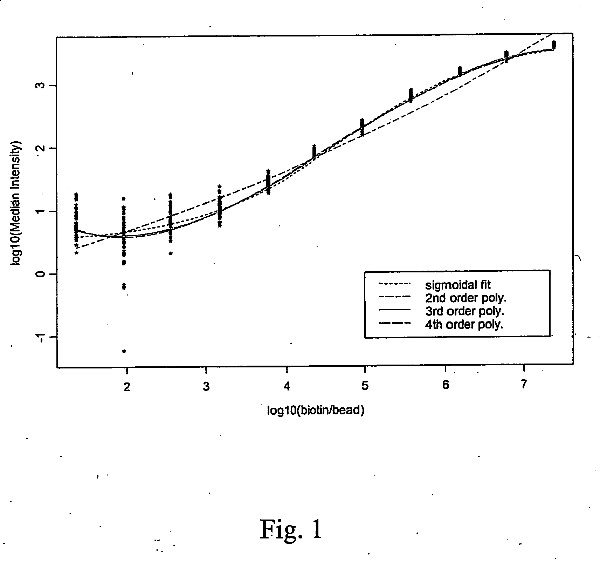

Four functions were proposed to fit the log of the median intensity as a function of the log of the biotin density. Second, third, and fourth order polynomials and a sigmoid were chosen for the functional forms of the data models. The functional form, best-fit parameters, and $r^2$ for the proposed models are presented in Table 5 and plotted in Figure 1 along with the raw data.

Table 5

| Functional Form | Best-Fit Parameters | $r^2$ |
|---|---|---|
| $$\log_{10}(I_{median}) = C_0 + C_1 \log_{10}(\delta_{biotin}) + C_2 [\log_{10}(\delta_{biotin})]^2$$ | $C_0 = -0.04$ <br> $C_1 = 0.29$ <br> $C_2 = 0.03$ | 0.946 |
| $$\log_{10}(I_{median}) = C_0 + C_1 \log_{10}(\delta_{biotin}) + C_2 [\log_{10}(\delta_{biotin})]^2 + C_3 [\log_{10}(\delta_{biotin})]^3$$ | $C_0 = 1.88$ <br> $C_1 = -1.48$ <br> $C_2 = 0.49$ <br> $C_3 = -0.03$ | 0.973 |
| $$\log_{10}(I_{median}) = C_0 + C_1 \log_{10}(\delta_{biotin}) + C_2 [\log_{10}(\delta_{biotin})]^2 + C_3 [\log_{10}(\delta_{biotin})]^3 + C_4 [\log_{10}(\delta_{biotin})]^4$$ | $C_0 = 2.34$ <br> $C_1 = -2.05$ <br> $C_2 = 0.72$ <br> $C_3 = -0.07$ <br> $C_4 = 0.002$ | 0.973 |
| $$\log_{10}(I_{median}) = C_0 + \frac{(C_1 - C_0)}{1 + 10^{C_3 [\log_{10}(\delta_{biotin}) - C_2]}}$$ | $C_0 = 0.50$ <br> $C_1 = 3.67$ <br> $C_2 = 4.73$ <br> $C_3 = 0.47$ | 0.970 |

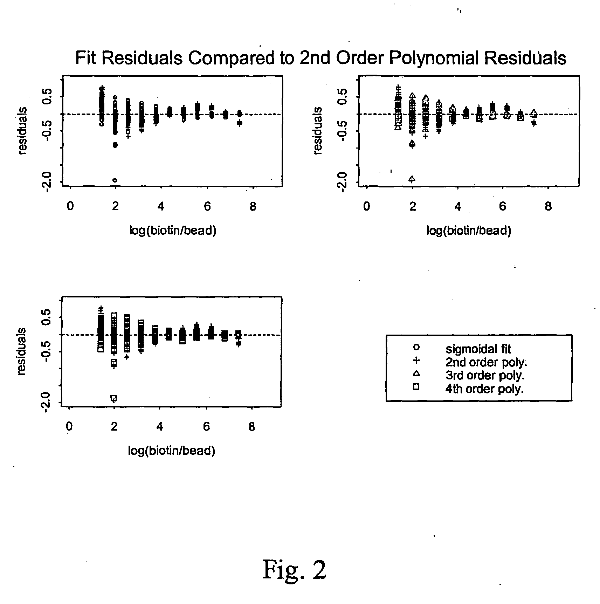

By inspection, the second order polynomial fit is the poorest and the other models are fairly close to each other. In order to better view the quality of these fits, the residuals for each fit are presented in Figure 2. The residuals for the third order polynomial, fourth order polynomial, and sigmoid are presented in separate graphs with the residuals for the second order polynomial repeated in each graph for reference.

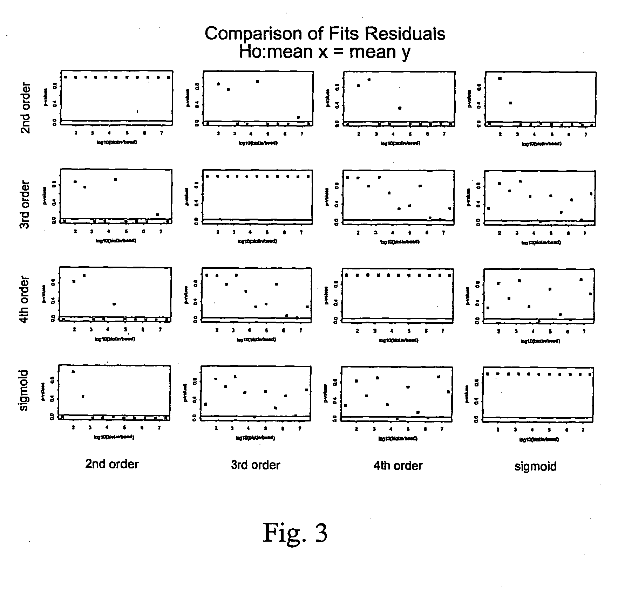

To quantify the quality of the data models, Student's t-test at a confidence level of 0.95 was used to check for a statistically significant difference in the residuals at each level of biotin density. This method was employed to allow comparison of the fits at discrete biotin levels. A pooled variance approach will not allow the difference in fits as a function of biotin density to be seen. The p-values for each comparison (Ho:$\mu_x$-$\mu_y$ = 0) are presented in Figure 3 as a matrix of plots where $\mu_x$ refers to the mean of the fit labeled along the x-axis of Figure 3 and $\mu_y$ refers to the mean of the fit labeled along the $\gamma$-axis of the Figure 3. Each individual graph plots the p-value vs. biotin density with the solid line representing the 95% confidence limit. Points below the line represent a statistically significant difference at that biotin density for fit 'x' compared to fit 'y'. Figure 3 illustrates that the third order polynomial, fourth order polynomial, and sigmoidal fit residuals are all different from the second order polynomial fit residuals and indistinguishable from each other at most biotin levels.

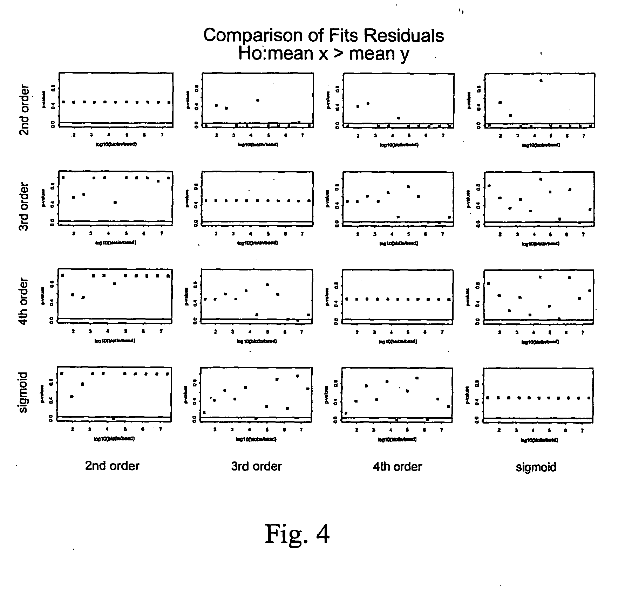

Figure 4 presents the results for the one-sided t-test (Ho:$\mu_x$-$\mu_y$ > 0) in the same format as Figure 3. For points below the 95% confidence limit, we reject the null hypothesis that the mean of the residuals for the fit labeled on the x-axis is greater than the mean of the residuals of the fit labeled on the $\gamma$-axis. Here, we show that, not surprisingly, the third order polynomial, fourth order polynomial, and sigmoidal fit residuals are all less than the second order polynomial fit residuals at most biotin levels. Since we cannot distinguish the third order polynomial, fourth order polynomial, and sigmoidal fit residuals from each other, neither can we state which of these fits has lower error.

Example 7: Comparison of analyte determination with and without use of a titration curve

Cells in culture were treated with different compounds at different concentrations to generate 15 samples. Following treatment, the cells were lysed and total RNA was extracted. The total RNA (100 ng) was then T7-amplified by a modification of the Eberwine protocol. The samples were analyzed individually using a mixture of sample beads and control beads which were present in every well. The samples were hybridized to the beads, washed, stained with the streptavidin Q655 reporter, washed and scanned on the Mosaic instrument (Quantum Dot Corporation; Hayward, CA).

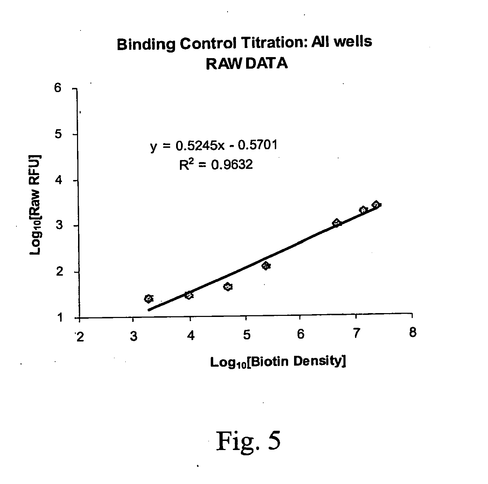

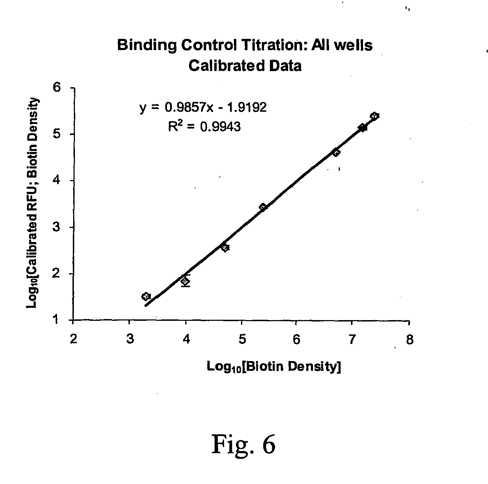

The raw data for the calibration curves for each of the 15 samples was plotted, comparing $\text{Log}_{10}$[Raw RFU] against $\text{Log}_{10}$[biotin/bead] (shown in Figure 5). The calibrated data for each of the calibration curves was plotted (shown in Figure 6), comparing the $\text{Log}_{10}$[Calibrated RFU] against the $\text{Log}_{10}$[biotin/bead].

The data was fit using a linear algorithm to give an equation of y = 0.581x - 1.467. The slope of the raw signal (RFU) as a function of the biotin density/bead was observed to be about 0.52 or only about a 1X increase in signal for a 2X increase in biotin density. For a 4-log range of biotin density, the observed raw signal dynamic range was observed to be 1.95 logs. When the raw calibration curve data was calibrated, the slope became about 0.99 and the signal dynamic range was observed to be 3.9 logs. Consequently, the calibrated RFU was observed to more closely reflect the changes in biotin density and the compressed signal dynamic range in the raw data was corrected to more closely reflect the range of biotin densities.

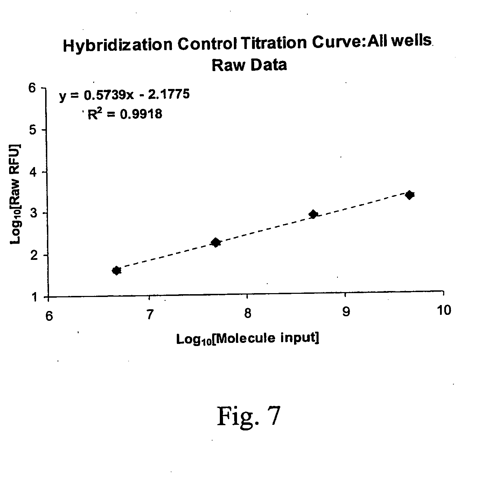

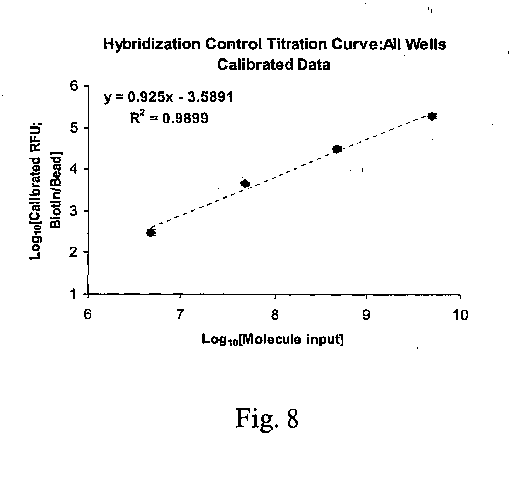

Next, the hybridization control beads in every well were used to evaluate the effect of the calibration. The biotin density titration (calibration) curve was used to calibrate the raw data. The raw data was plotted, comparing $\text{Log}_{10}$[Raw RFU] against $\text{Log}_{10}$[molecule input] (shown in Figure 7). The data was fit using a linear algorithm to give an equation of y = 0.5739x - 2.1775, with an $R^2$ value of 0.99. Similarly, the $\text{Log}_{10}$[Calibrated RFU; biotins per bead] against $\text{Log}_{10}$[molecule input] is shown in Figure 8. The data was fit using a linear algorithm to give an equation of y = 0.925x - 3.5891, with an $R^2$ value of 0.99 (shown in Figure 8).

Given the known copy number input of the biotinylated oligos used in the hybridization control titration curve, the expected slope was 1 and the signal dynamic range was expected to be 3 logs. The raw data produced a slope of about 0.57 and a signal dynamic range of about 1.7 logs. This again means that the raw data was compressed in both change and range relative to the known inputs. Furthermore, the values obtained for the slope and signal dynamic range for the raw data from the hybridization controls was observed to be very similar to those observed for the raw data from the calibration curve. Calibration of the raw data now resulted in a hybridization titration curve slope of 0.93 and a dynamic signal range of 2.8 logs. The end result was that the change in signal more closely replicated the change of input into the hybridization and the signal dynamic range more closely replicated the input dynamic range. The similarity of the slopes and signal dynamic range for the hybridization controls and the biotin binding calibration curve indicated that the compression in the hybridization signal was a result of the biotin binding to the bead.

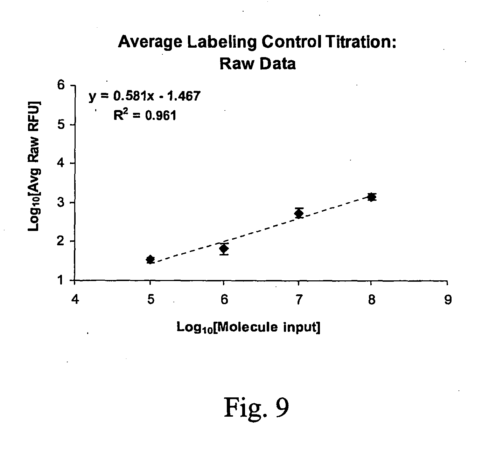

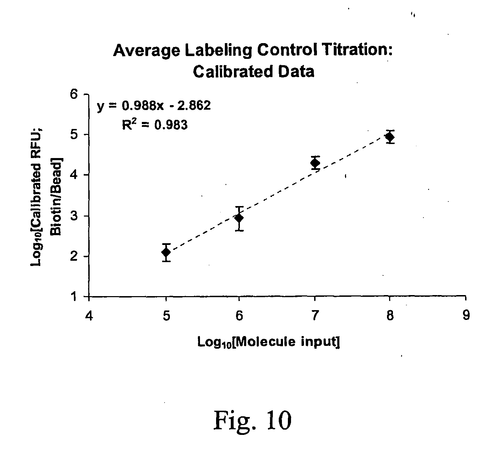

The RNA spike titration curve consisted of RNAs that were spiked into each sample prior to the T7 amplification step at known copy number inputs. The biotin density titration (calibration) curve was used to calibrate the raw data. The raw data was plotted, comparing $\text{Log}_{10}$[Raw RFU] against $\text{Log}_{10}$[molecule input] (shown in Figure 9). The data was fit using a linear algorithm to give an equation of y = 0.581x - 1.467, with an $R^2$ value of 0.96. Similarly, the $\text{Log}_{10}$[Calibrated RFU; biotins per bead] against $\text{Log}_{10}$[molecule input] is shown in Figure 8. The data was fit using a linear algorithm to give an equation of y = 0.988x - 2.862, with an $R^2$ value of 0.98 (shown in Figure 10).

Given the known copy number input of the RNA transcripts spiked into the samples to form the RNA titration curve, the expected slope was 1 and the signal dynamic range was expected to be 3 logs. The raw data produced a slope of about 0.58 and a signal dynamic range of about 1.6 logs. This again means that the raw data was compressed in both change and range relative to the known inputs into the T7 amplification. Furthermore, the values obtained for the slope and signal dynamic range for the raw data from the hybridization controls was observed to be very similar to those observed for the raw data from the calibration curve. Calibration of the raw data now resulted in a hybridization titration curve slope of 0.99 and a dynamic signal range of 2.8 logs. The end result was that the change in signal more closely replicated the change of input into the hybridization and the signal dynamic range more closely replicated the input dynamic range. The similarity of the slopes and signal dynamic range for the RNA spike titration controls, the hybridization controls and the biotin binding calibration curve indicated that the compression in the hybridization signal was a result of the biotin binding to the bead.

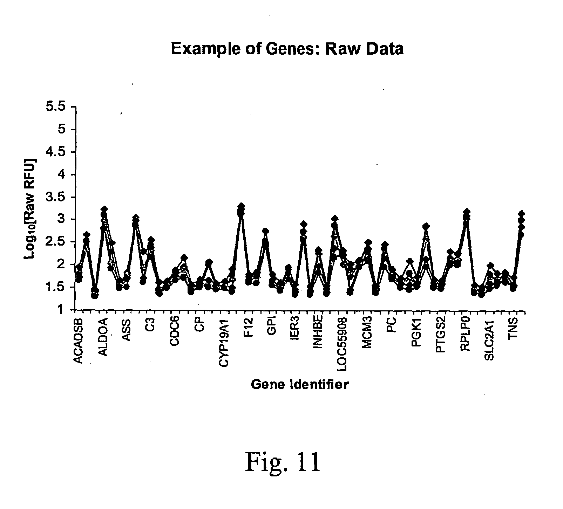

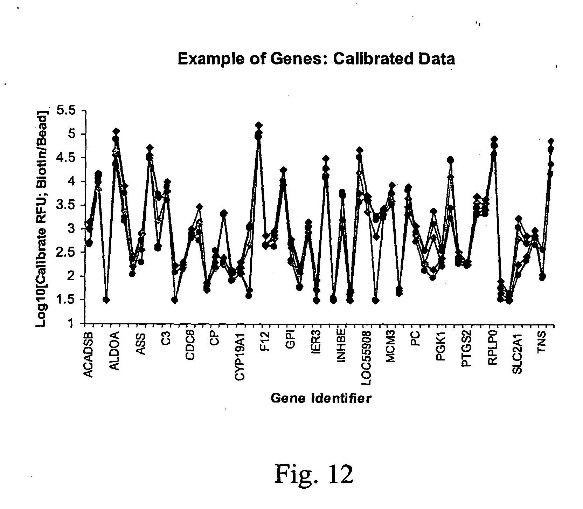

In the same wells as the control bead sets were beads for analysis of expression levels of genes endogenous to the cells. The expression level for these genes was evaluated with the raw signal (Figure 11). The biotin density titration (calibration) curve was used to calibrate the raw data as was described above for the controls (Figure 12).

Both the signal dynamic range and the changes in expression were compressed with the raw data. The calibrated data demonstrated a greater signal dynamic range and changes in gene expression were more easily observed.

**Claims**

1. A method of determining the concentration of at least one analyte in a multiplexed assay, the method comprising:

   providing a mixture comprising a set of sample beads and a set of control beads, wherein the set of sample beads comprise a first coding moiety and a first capture moiety that selectively binds to the at least one analyte, and wherein the set of control beads comprise a second coding moiety at a fixed concentration in the plurality of beads and a calibration moiety present at a range of concentrations in the plurality of beads;
   contracting the mixture with a sample suspected of containing the at least one analyte;
   obtaining signal from the first coding moiety;
   obtaining signal from the second coding moiety;
   obtaining signal from the calibration moiety;
   preparing a titration curve from the signal obtained from the calibration moiety; and
   using the titration curve and the image to determine the concentration of the at least one analyte.

2. The method of claim 1, wherein the signal obtained from the first coding moiety, the signal obtained from the second coding moiety, and the signal obtained from the calibration moiety are obtained simultaneously.

3. The method of claim 1, wherein the preparing a titration curve step comprises analyzing the signal obtained from the calibration moiety using a first order polynomial, second order polynomial, third order polynomial, a fourth order polynomial, or a sigmoidal fit

4. The method of claim 1, wherein the first coding moiety is at least one semiconductor nanocrystal.

5. The method of claim 1, wherein the second coding moiety is at least one semiconductor nanocrystal.

6. The method of claim 1, wherein:

   the first coding moiety is at least one semiconductor nanocrystal; and
   the second coding moiety is at least one semiconductor nanocrystal.

7. The method of claim 1, wherein the calibration moiety is a directly detectable moiety.

8. The method of claim 1, wherein the calibration moiety is an indirectly detectable moiety.

9. The method of claim 1, wherein the calibration moiety is a biotinylated compound.

10. The method of claim 1, wherein the calibration moiety is a biotinylated oligonucleotide.

11. The method of claim 1, further comprising contacting the mixture with labeled streptavidin before the simultaneously imaging step.

12. The method of claim 1, further comprising contacting the mixture with streptavidin labeled with at least one semiconductor nanocrystal before the simultaneously imaging step.

13. The method of claim 1, wherein the set of sample beads are polymer beads.

14. The method of claim 1, wherein the set of sample beads are polystyrene beads.

15. The method of claim 1, wherein the set of control beads are polymer beads.

16. The method of claim 1, wherein the set of control beads are polystyrene beads.

**17.** The method of claim 1, wherein the at least one analyte is DNA, RNA, PNA, a protein, an antibody, a ligand, a receptor, a lipid, or a polysaccharide.

**18.** The method of claim 1, wherein the at least one analyte is DNA.

**19.** The method of claim 1, wherein the at least one analyte is biotinylated DNA.

**20.** The method of claim 1, wherein the first set of beads comprise a first semiconductor nanocrystal and an oligonucleotide that selectively binds to the at least one analyte, and wherein the second set of beads comprise a second semiconductor nanocrystal and a biotinylated oligonucleotide; the method comprising : contacting the nixture with a sample suspected of containing the at least one analyte;
contacting the mixture with streptavidin labeled with a third semiconductor nanocrystal;
simultaneously imaging the mixture to produce an image, wherein the image comprises signal obtained from the first semiconductor nanocrystal, signal obtained from the second semiconductor nanocrystal, and signal obtained from the third semiconductor nanocrystal;
preparing a titration curve from the signal obtained from the third semiconductor nanocrystal; and
using the titration curve and the image to determine the concentration of the at least one analyte.

**Patentansprüche**

**1.** Verfahren zum Bestimmen der Konzentration wenigstens eines Analyts in einem Multiplex-Test, wobei das Verfahren umfasst:

Bereitstellen einer Mischung eines Probenkügelchensatzes und eines Kontrollkügelchensatzes, wobei der Satz der Probenkügelchen eine erste kodierende Einheit und eine erste aufnehmende Einheit, die selektiv an den wenigstens einen Analyten bindet, und wobei der Satz der Kontrollkügelchen eine zweite kodierende Einheit mit einer festen Konzentration in der Mehrzahl der Kügelchen und eine Kalibrationseinheit umfasst, die in einem Bereich von Konzentrationen in der Vielzahl von Kügelchen vorliegt;
in Kontakt bringen der Mischung mit einer Probe, vor der angenommen wird, dass sie den wenigstens einen Analyten enthält;
Erhalten eines Signals von der ersten kodierenden Einheit;
Erhalten eines Signals von der zweiten kodierenden Einheit;
Erhalten eines Signals von der Kalibrationseinheit;
Erstellen einer Titrationskurve aus dem von der Kalibrationseinheit erhaltenen Signal und
Verwenden der Titrationskurve und des Bildes, um die Konzentration des wenigstens einen Analyten zu bestimmen.

**2.** Verfahren gemäß Anspruch 1, worin das von der ersten kodierenden Einheit erhaltene Signal, das von der zweiten kodierenden Einheit erhaltene Signal und das von der Kalibrationseinheit erhaltene Signal gleichzeitig erhalten werden.

**3.** Verfahren gemäß Anspruch 1, worin der Schritt des Erstellens einer Titrationskurve das Analysieren des von der Kalibrationseinheit erhaltenen Signals unter Verwendung einer Anpassung an ein Polynom erster Ordnung, ein Polynom zweiter Ordnung, ein Polynom dritter Ordnung, ein Polynom vierter Ordnung oder einer sigmoiden Anpassung umfasst.

**4.** Verfahren gemäß Anspruch 1, worin die erste kodierende Einheit wenigstens ein Halbleiter-Nanokristall ist.

**5.** Verfahren gemäß Anspruch 1, worin die zweite kodierende Einheit wenigstens ein Halbleiter-Nanokristall ist.

**6.** Verfahren gemäß Anspruch 1, worin
die erste kodierende Einheit wenigstens ein Halbleiter-Nanokristall ist und
die zweite kodierende Einheit wenigstens ein Halbleiter-Nanokristall ist.

**7.** Verfahren gemäß Anspruch 1, worin die Kalibrationseinheit eine direkt detektierbare Einheit ist.

**8.** Verfahren gemäß Anspruch 1, worin die Kalibrationseinheit eine indirekt detektierbare Einheit ist.

9. Verfahren gemäß Anspruch 1, worin die Kalibrationseinheit eine biotinylierte Verbindung ist.

10. Verfahren gemäß Anspruch 1, worin die Kalibrationseinheit ein biotinyliertes Oligonukleotid ist.

11. Verfahren gemäß Anspruch 1, ferner umfassend das in Kontakt bringen der Mischung mit markiertem Streptavidin vor dem Schritt des gleichzeitigen Bildgebens.

12. Verfahren gemäß Anspruch 1, ferner umfassend das in Kontakt bringen der Mischung mit Streptavidin, das mit wenigstens einem Halbleiter-Nanokristall markiert ist, vor dem Schritt des gleichzeitigen Bildgebens.

13. Verfahren gemäß Anspruch 1, worin der Satz der Probenkügelchen Polymerkügelchen ist.

14. Verfahren gemäß Anspruch 1, worin der Satz der Probenkügelchen Polystyrolkügelchen ist.

15. Verfahren gemäß Anspruch 1, worin der Satz der Kontrollkügelchen Polymerkügelchen ist.

16. Verfahren gemäß Anspruch 1, worin der Satz der Kontrollkügelchen Polystyrolkügelchen ist.

17. Verfahren gemäß Anspruch 1, worin der wenigstens eine Analyt DNA, RNA, PNA, ein Protein, ein Antikörper, ein Ligand, ein Rezeptor, ein Lipid oder ein Polysaccharid ist.

18. Verfahren gemäß Anspruch 1, worin der wenigstens eine Analyt DNA ist.

19. Verfahren gemäß Anspruch 1, worin der wenigstens eine Analyt biotinylierte DNA ist.

20. Verfahren gemäß Anspruch 1, worin der erste Satz der Kügelchen einen ersten Halbleiter-Nanokristall und ein Oligonukleotid, das selektiv an den wenigstens einen Analyt bindet, umfasst, und worin der zweite Satz Kügelchen einen zweiten Halbleiter-Nanokristall und ein biotinyliertes Oligonukleotid umfasst, wobei das Verfahren umfasst:

in Kontakt bringen der Mischung mit einer Probe, vorn der vermutet wird, dass sie den wenigstens einen Analyten enthält;
in Kontakt bringen der Mischung mit Streptavidin, das mit einem dritten Halbleiter-Nanokristall markiert ist;
gleichzeitiges Bildgeben der Mischung, um ein Bild zu erstellen, worin das Bild ein von dem ersten Halbleiter-Nanokristall erhaltenes Signal, ein von dem zweiten Halbleiter-Nanokristall erhaltenes Signal und ein von dem dritten Halbleiter-Nanokristall erhaltenes Signal umfasst;
Erstellen einer Titrationskurve aus dem von dem dritten Halbleiter-Nanokristall erhaltenen Signal und
Verwenden der Titrationskurve und des Bilds, um die Konzentration des wenigstens einen Analyten zu bestimmen.

**Revendications**

1. Procédé de détermination de la concentration d'au moins une substance à analyser dans un dosage multiple, le procédé comprenant :

la fourniture d'un mélange comprenant un ensemble de billes d'échantillonnage et un ensemble de billes de contrôle, dans lequel l'ensemble de billes d'échantillonnage comporte un premier fragment de codage et un premier fragment de capture qui se lie sélectivement à ladite au moins une substance à analyser, et dans lequel l'ensemble de billes de contrôle comporte un second fragment de codage à une concentration fixe dans la pluralité de billes et un fragment d'étalonnage présent à une plage de concentrations dans la pluralité de billes ;
la mise en contact du mélange avec un échantillon suspecté de contenir ladite au moins une substance à analyser ;
l'obtention d'un signal à partir du premier fragment de codage ;
l'obtention d'un signal à partir du second fragment de codage ;
l'obtention d'un signal à partir du fragment d'étalonnage ;
la préparation d'une courbe de titrage à partir du signal obtenu à partir du fragment d'étalonnage ; et
l'utilisation de la courbe de titrage et de l'image afin de déterminer la concentration de ladite au moins une substance à analyser.

**EP 1 836 497 B1**

2. Procédé selon la revendication 1, dans lequel le signal obtenu à partir du premier fragment de codage, le signal obtenu à partir du second fragment de codage et le signal obtenu à partir du fragment d'étalonnage sont obtenus simultanément.

3. Procédé selon la revendication 1, dans lequel l'étape de préparation d'une courbe de titrage comprend l'analyse du signal obtenu à partir du fragment d'étalonnage au moyen d'un polynôme du premier ordre, d'un polynôme du deuxième ordre, d'un polynôme du troisième ordre, d'un polynôme du quatrième ordre ou d'un ajustement sigmoïdal.

4. Procédé selon la revendication 1, dans lequel le premier fragment de codage est au moins un nanocristal semi-conducteur.

5. Procédé selon la revendication 1, dans lequel le second fragment de codage est au moins un nanocristal semi-conducteur.

6. Procédé selon la revendication 1, dans lequel :

le premier fragment de codage est au moins un nanocristal semi-conducteur ; et
le second fragment de codage est au moins un nanocristal semi-conducteur.

7. Procédé selon la revendication 1, dans lequel le fragment d'étalonnage est un fragment directement détectable.

8. Procédé selon la revendication 1, dans lequel le fragment d'étalonnage est un fragment indirectement détectable.

9. Procédé selon la revendication 1, dans lequel le fragment d'étalonnage est un composé biotinylé.

10. Procédé selon la revendication 1, dans lequel le fragment d'étalonnage est un oligonucléotidebiotinylé.

11. Procédé selon la revendication 1, comprenant en outre la mise en contact du mélange avec de la streptavidine marquée, préalablement à l'étape de formation d'image simultanée.

12. Procédé selon la revendication 1, comprenant en outre la mise en contact du mélange contenant de la streptavidine marquée avec au moins un nanocristal semi-conducteur, préalablement à l'étape de formation d'image simultanée.

13. Procédé selon la revendication 1, dans lequel l'ensemble de billes d'échantillonnage représente des billes de polymère.

14. Procédé selon la revendication 1, dans lequel l'ensemble de billes d'échantillonnage représente des billes de polystyrène.

15. Procédé selon la revendication 1, dans lequel l'ensemble de billes de contrôle représente des billes de polymère.

16. Procédé selon la revendication 1, dans lequel l'ensemble de billes de contrôle représente des billes de polystyrène.

17. Procédé selon la revendication 1, dans lequel ladite au moins une substance à analyser est de l'ADN, de l'ARN, de l'APN, une protéine, un anticorps, un ligand, un récepteur, un lipide ou un polysaccharide.

18. Procédé selon la revendication 1, dans lequel ladite au moins une substance à analyser est de l'ADN.

19. Procédé selon la revendication 1, dans lequel ladite au moins une substance à analyser est de l'ADN biotinylé.

20. Procédé selon la revendication 1,
dans lequel le premier ensemble de billes comporte un premier nanocristal semi-conducteur et un oligonucléotide qui se lie sélectivement à ladite au moins une substance à analyser, et dans lequel le second ensemble de billes comporte un deuxième nanocristal semi-conducteur et un oligonucléotidebiotinylé ;
le procédé comprenant :

la mise en contact du mélange avec un échantillon suspecté de contenir ladite au moins une substance à analyser ;

14

la mise en contact du mélange contenant de la streptavidine marquée avec un troisième nanocristal semi-conducteur ;

l'imagerie simultanée du mélange en vue de produire une image, dans laquelle l'image comprend le signal obtenu à partir du premier nanocristal semi-conducteur, le signal obtenu à partir du deuxième nanocristal semi-conducteur et le signal obtenu à partir du troisième nanocristal semi-conducteur ;

la préparation d'une courbe de titrage à partir du signal obtenu à partir du troisième nanocristal semi-conducteur ; et

l'utilisation de la courbe de titrage et de l'image en vue de déterminer la concentration de ladite au moins une substance à analyser.

Fig. 1

Fig. 2

Fig. 3

Fig. 4

Fig. 5

Fig. 6

**Hybridization Control Titration Curve:All wells Raw Data**

$y = 0.5739x - 2.1775$

$R^2 = 0.9918$

Fig. 7

**Hybridization Control Titration Curve:All Wells Calibrated Data**

$y = 0.925x - 3.5891$
$R^2 = 0.9899$

Fig. 8

**Average Labeling Control Titration:**
**Raw Data**

Fig. 9

**Average Labeling Control Titration: Calibrated Data**

$y = 0.988x - 2.862$

$R^2 = 0.983$

Fig. 10

**Example of Genes: Raw Data**

Fig. 11

Fig. 12

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2002187515 A1
- US 2003165951 A1
- US 6207299 B
- US 6322901 B
- US 6576291 B
- US 6815064 B
- US 20030097976 A
- US 6649139 B
- US 6479146 B

- WO 0077281 A
- WO 0151196 A
- WO 9947252 A
- US 6548171 B1
- US 6680211 B2
- US 5514545 A
- US 5545522 A
- US 5716785 A
- US 5891636 A

**Non-patent literature cited in the description**

- **QU et al.** Alternative Routes toward High Quality CdSe Nanocrystals. *Nano Lett.,* 2001, vol. 1 (6), 333-337

- **PARK et al.** *Langmuir,* 2002, vol. 18, 9600-9604